# EUROPÄISCHE PATENTANMELDUNG

(11) **EP 0 911 324 A1**
(43) Veröffentlichungstag der Anmeldung: **28.04.1999**
(21) Anmeldenummer: 98118247.0
(22) Anmeldetag: 25.09.1998
(51) Int. Cl.: C07D 233/72, C07D 295/18, C07C 69/608

(54) **Verfahren zur Herstellung von chiralen Bernsteinsäurederivaten**

(30) Priorität: 03.10.1997 EP 97117191
(71) Anmelder: F. HOFFMANN-LA ROCHE AG, 4070 Basel (CH)
(72) Erfinder: Hilpert, Hans, 4153 Reinach (CH)
(74) Vertreter: Löschner, Thomas

(57) **Zusammenfassung**

Die vorliegende Erfindung betrifft ein Verfahren zur Herstellung von chiralen Bernsteinsäurederivaten der Formel (I) 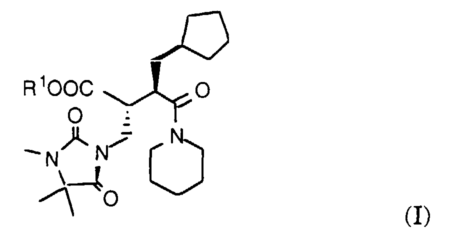 worin R¹ (C₁-C₆)Alkyl oder Benzyl ist, und die darin verwendeten neuen Zwischenverbindungen.

## Beschreibung

Die vorliegende Erfindung betrifft ein Verfahren zur Herstellung von chiralen Bernsteinsäurederivaten der Formel (I) 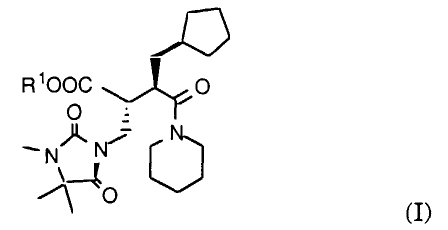 worin R¹ (C₁-C₆)Alkyl oder Benzyl ist, dadurch gekennzeichnet, dass eine Verbindung der Formel (II) 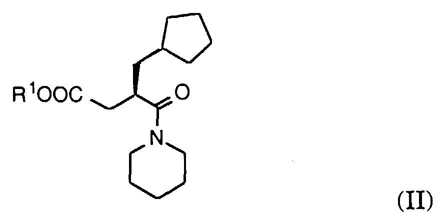 worin R¹ die vorstehend genannte Bedeutung hat,
mit einem Halogenhydantoin der Formel (III) 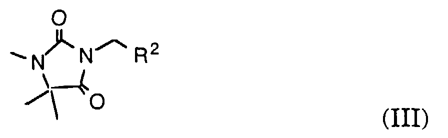 worin R² Halogen ist,
in Gegenwart einer starken, enolatbildenden Kaliumbase umgesetzt wird.

Unter Halogen wird im folgenden Chlor, Brom und Jod verstanden. (C₁-C₆)Alkyl bedeutet eine gradkettige oder verzweigte Alkylgruppe mit 1 bis 6 C-Atomen, wie z.B. Methyl, Ethyl, Propyl, Butyl, Pentyl, Hexyl oder iso-Propyl bzw. tert.Butyl. Vorzugsweise ist R¹ ein (C₁-C₄)Alkyl. Für den Begriff (C₁-C₆)Alkoxy gelten ebenfalls die vorstehenden Definitionen bezüglich des an den Sauerstoff gebundenen Alkylteils.

Verbindungen der Formel (I) sind in der europäischen Patentanmeldung mit der Publikationsnummer EP 0 816 341 A1 beschrieben. Verbindungen der Formel (I) sind wertvolle Zwischenverbindungen bei der Synthese der pharmakologisch wirksamen Verbindung (X).

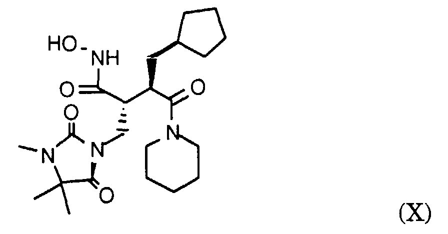

Die Alkylierung von Verbindungen der Formel II mit dem Halogenmethylhydantoin (III) erfolgt in Gegenwart einer starken Base in einem Lösungsmittel wie einem Ether, vorzugsweise THF, bei einer Temperatur von -100° bis 22°, vorzugsweise -60°C.

Die Stereoselektivität des neu gebildeten Stereozentrums hängt aber sehr stark von der Natur des Kations der Base ab. Lithiumbasen, wie z.B. LDA, erzeugen syn-Selektivität (Verhältnis bis 90:10). Andererseits sind Natriumbasen, wie z.B. NaN(TMS)₂ unspezifisch (1:1-Gemisch). Die syn-Selektivität mit LDA ist bereits von R. Becket et al. in Synlett 137, Feb. 1993 bei der Verwendung von Bernsteinsäurederivaten mit einer Estergruppe und einer freien Säuregruppe beschrieben worden. Unerwarteterweise wurde nun gefunden, dass im Falle eines Säureamids und der Verwendung von starken, enolatbildenden Kaliumbasen wie z.B. KN(TMS)₂ oder C₁-C₆-Alkoxykaliumbasen, wie z.B. Kalium-tert.-butylat, die für die Herstellung von Verbindungen der Formel (I) benötigte anti-Selektivität erhalten wird. Vorzugsweise wird bei der Herstellung von Verbindungen der Formel (I) KN(TMS)₂ verwendet. Damit wird eine anti-Selektivität im Verhältnis 90:10 erzielt. Das Gemisch der Diastereomeren kann durch Chromatographie an Kieselgel mit geeigneten Lösungsmitteln wie etwa Hexan/Essigester getrennt werden. Als Ester wird vorzugsweise der tert-butyl Ester in einer Verbindung der Formel (II) verwendet. Das Halogenhydantoin (III) für die Reaktion mit einer Verbindung (II) kann durch Halogenmethylierung von 1,5,5-Trimethylhydantoin erhalten werden. Dazu wird zweckmässig 1,5,5-Trimethylhydantoin mit einem Halogenwasserstoff in Essigsäure bei einer Temperatur zwischen 20° und 100°, vorzugsweise bei etwa 80°, umgesetzt. Das Trimethylhydantoin ist nach an sich bekannten Methoden erhältlich (H. Heimgartner et al., Helv. Chim. Acta 75, 1251 (1992)). Halogen bedeutet dabei Chlor, Brom oder Jod. Vorzugsweise ist das Halogen Brom.

In Zusammenhang mit der Herstellung von Verbindungen der Formel (I) via Verbindungen der Formel (II) betrifft die vorliegende Erfindung auch ein Verfahren zur Herstellung von Bernsteinsäurederivaten der Formel (I), dadurch gekennzeichnet, dass
(a) eine Verbindung der Formel (IV) 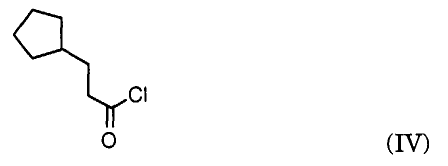 mit (S)-4-Benzyl-2-oxazolidinon zu (S)-3-(3-Cyclopentyl-1-oxopropyl)-4-(phenylmethyl)-2-oxazolidinon (V) umgesetzt wird,
(b) das erhaltene Produkt (V) mit einer Verbindung der Formel (VI) 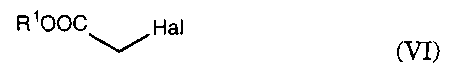 worin R¹ (C₁-C₆)Alkyl oder Benzyl und Hal Halogen bedeuten, zu einer Verbindung der Formel (VII) 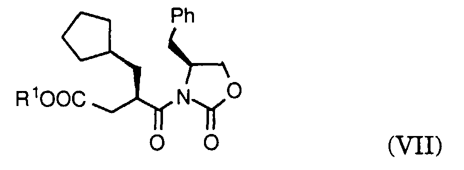 umgesetzt wird,
c) aus Verbindung (VII) unter Abspaltung von (S)-4-Benzyl-2-oxazolidinon eine Verbindung mit der Formel (VIII) erhält, 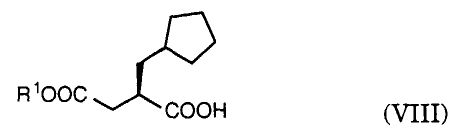
d) durch Umsetzung der Verbindung (VIII) mit Piperidin eine Verbindung der Formel (II) 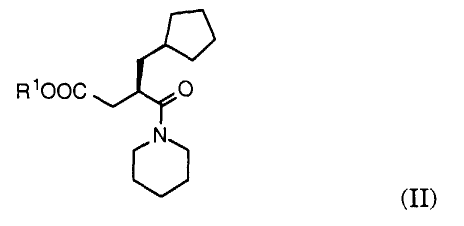 erhalten wird, und
e) die so erhaltene Verbindung der Formel (II) mit einem Halogenhydantoin der Formel (III) 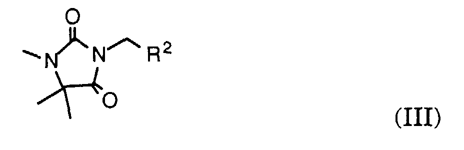 worin R² Halogen ist, umgesetzt wird.

Für die Durchführung von Schritt e) wird auf die vorstehende Beschreibung für die Umsetzung von Verbindungen der Formel (II) mit solchen der Formel (III) verwiesen.

Die Acylierung von (S)-4-Benzyl-2-oxazolidinon (käuflich erhältlich oder herstellbar gemäss M. Sudharshan, P.G. Hultin, Synlett, 171 (1997)) mit Cyclopentyl-propionsäurechlorid (IV) (Barret et al., J. Chemical Society 1065 (1935)) gemäss Schritt a) erfolgt nach an sich bekannter Methode mit einer Base, z.B. NaH, LDA, LiN (TMS)₂, oder einer Alkyllithium Verbindung, bevorzugt BuLi, in einem Lösungsmittel wie einem Ether, bevorzugt THF, bei einer Temperatur von -80° bis 22°, vorzugsweise -45°. Für die Bildung der alkylierten Verbindungen (VII) kann isoliertes oder, zweckmässigerweise, in Lösung verbleibendes (S)-3-(3-Cyclopentyl-1-oxopropyl)-4-(phenylmethyl)-2-oxazolidinon verwendet werden. Die Alkylierung erfolgt mit einem Halogen-Essigsäureester, bevorzugt, Bromessigsäure-tert-butylester in Gegenwart einer Base, z.B. LiN(TMS)₂ oder bevorzugt LDA in einem oben genannten Lösungsmittel, vorzugsweise THF, bei -80° bis 22°, vorzugsweise -45°. Das gebildete Produkt VII kann aus dem Reaktionsmedium durch Kristallisation durch Zusatz von einem Alkan, bevorzugt Hexan, oder durch Chromatographie in hoher optischer Reinheit (de>99.9%) erhalten werden.

Die Halogen-Essigsäureester sind kommerziell oder nach an sich bekannten Methoden durch Veresterung der Halogenessigsäurederivate erhältlich.

Die Abspaltung des chiralen Hilfsreagenzens aus Verbindungen der Formel VII zur Säure VIII und dem (S)-4-Benzyl-2-oxazolidinon gemäss Schritt c) kann nach an sich bekannter Methode mit Wasserstoffperoxid und LiOH in einem Ether, wie z.B. Tetrahydrofuran erfolgen. Das dabei auftretende Peroxid des THF stellt ein nicht unerhebliches Sicherheitsrisiko dar. Unerwarteterweise wurde nun gefunden, dass die Reaktion quantitativ verläuft, wenn die billigere Natronlauge in einem Gemisch von Wasser und einem Alkohol, bevorzugt Isopropanol, bei einer Temperatur von -10° - 22°, bevorzugt 0°, verwendet wird. Das dabei auftretende (S-)-4-Benzyl-2-oxazolidinon kristallisiert praktisch quantitativ aus der wässrigen Phase aus.

Die Amidbildung der Säure VIII mit Piperidin in Schritt d) kann nach an sich bekannten Kopplungsmethoden, wie z.B. via Säurechlorid, via gemischtes Anhydrid, via gemischtes Sulfonsäureanhydrid, oder bevorzugt, via einen Aktivester erfolgen. Dabei werden wasserziehende Mittel wie Carbodiimide, bevorzugt Dicyclohexylcarbodiimid in Gegenwart von stöchiometrischen oder katalytischen Mengen an Aktivestern bildenden Alkoholen, wie z.B. N-Hydroxysuccinimid, N-Hydroxybenzotriazol oder bevorzugt N-Hydroxy-2-pyridon, in einem Lösungsmittel wie einem Keton, z.B. Methylethylketon, oder einem Ether, z.B. tert-Butyl-methylether, oder einem Kohlenwasserstoff, z.B. Toluol, oder einem halogenierten Kohlenwasserstoff, z.B. Methylenchlorid oder einem Ester, bevorzugt Isopropylester, bei einer Temperatur von 0 bis 80°, bevorzugt 22°, eingesetzt.

Verbindungen der Formel (I) können zur Herstellung einer Verbindung der Formel (X) 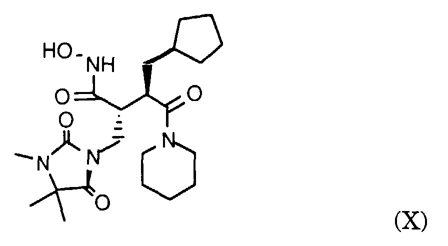 verwendet werden. Das Verfahren dafür ist dadurch gekennzeichnet, dass
a) eine Verbindung der Formel (I) 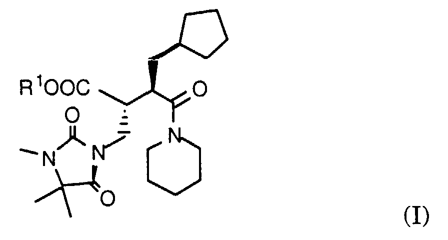 worin R¹ (C₁-C₆)Alkyl oder Benzyl bedeutet, gemäss der vorstehenden Beschreibung erhalten wird,
b) anschliessend durch Abspaltung der R¹ Gruppe die Verbindung mit der Formel (IX) 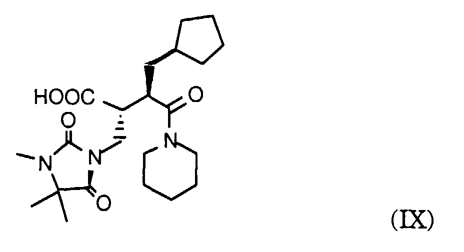 hergestellt wird, und
c) durch nachfolgende Einführung der Hydroxylamingruppe in der Verbindung der Formel (IX) die Verbindung der Formel (X) 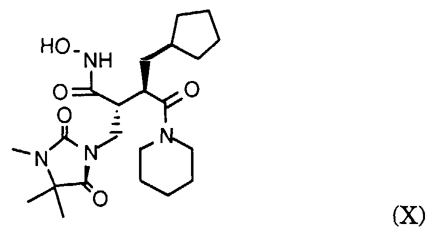 erhalten wird, oder
d) eine Verbindung der Formel (I), worin R¹ geradkettiges (C₁-C₆)Alkyl bedeutet,
wird mit einem mittels eines Alkylmagnesiumhalogenids aktivierten Benzylhydroxylaminhydrochlorids umgesetzt und anschliessend die Benzylgruppe hydrogenolytisch abgespalten, wobei ebenfalls Verbindung (X) erhalten wird.

Die Verbindung (X) ist bekannt und beispielsweise in EP 684 240 A1 beschrieben. Die Verbindung hat wertvolle pharmakologische Eigenschaften und kann daher zur Behandlung und Vorbeugung von Krankheiten wie etwa degenerativen Gelenkerkrankungen verwendet werden.

Die Hydrolyse einer Estergruppe in einer Verbindung der Formel I, in der R¹ geradkettiges oder verzweigtes (C₁-C₆)Alkyl, ausser tert.Butyl bzw. eine ähnlich sterisch anspruchsvolle Alkylgruppe, bedeutet, zur Verbindung IX gemäss Absatz b) erfolgt in Gegenwart eines Alkali- oder Erdalkalimetallhydroxids wie Barium-, Kalzium-, Natrium- oder Kaliumhydroxid, bevorzugt Kaliumhydroxid, in einem Lösungsmittel wie einem Alkohol, z.B. i-Propanol oder Wasser mit einem organischen Lösungsmittel wie einem Ether, z.B. tert-Butylmethylether, oder bevorzugt THF, bei einer Temperatur von 0 bis 100°, bevorzugt 30 bis 50°.

Die Abspaltung der tert-Butylgruppe oder einer sterisch ähnlich anspruchsvollen Alkylgruppe in einer Verbindung der Formel I, zur Verbindung IX gemäss Absatz b) erfolgt in Gegenwart einer Mineralsäure, wie z.B. wässriger Phosphor- oder Schwefelsäure, bevorzugt Salzsäure oder Bromwasserstoffsäure und einer organischen Carbonsäure, bevorzugt Essigsäure bei einer Temperatur von 0 bis 100°, bevorzugt 0 - 22°. Statt in einer Carbonsäure kann die Abspaltung auch in einem Ester einer Carbonsäure oder einem Gemisch von Carbonsäure und Carbonsäureester erfolgen. Geeignete Carbonsäureester sind Essigsäuremethyl, -ethyl oder - isopropylester, bevorzugt Essigsäureethylester. Als bevorzugte Abspaltungsmethode wird HBr in Essigsäure verwendet. Weiterhin kann die Abspaltung mittels einer Säure auch in einem sonstigen geeigneten organischen Lösungsmittel erfolgen. Geeignete organische Lösungsmittel sind Methylenchlorid oder Toluol.

Die Debenzylierung der Verbindung (I), in der R¹ gleich Benzyl (Bz) ist, in Schritt b) zu Verbindung IX erfolgt in einem organischen Lösungsmittel unter Verwendung von Wasserstoff in Gegenwart eines Metallkatalysators. Geeignete Lösungsmittel sind C₁-C₆-Alkohole, bevorzugt Methanol oder Ethanol. Als Metallkatalysatoren können Platin oder Palladium verwendet werden, die sich zweckmässigerweise auf einem Trägermaterial wie Aluminiumoxid, Bariumsulfat oder Kohle befinden. Ein bevorzugter Katalysator ist Palladium auf Kohle oder Bariumsulfat. Temperatur und Druck sind nicht kritisch und können in einem weiten Bereich gewählt werden. Vorzugsweise wird die Hydrierung bei Raumtemperatur und 1-10 bar durchgeführt.

Die Einführung der Hydroxylamingruppe in Verbindung IX gemäss Absatz c) kann mittels O-Trimethylsilyl-hydroxylamin erfolgen mit an sich bekannten Aktivierungsmitteln wie Carbodiimiden, z.B. Dicyclohexylcarbodiimid oder einem Isocyanid, z.B. tert-Butylisocyanid, bevorzugt 2-Morpholino-ethylisocyanid in Gegenwart von stöchiometrischen oder katalytischen Mengen an Aktivestern bildenden Alkoholen, wie z.B. N-Hydroxysuccinimid, N-Hydroxybenzotriazol oder bevorzugt N-Hydroxy-2-pyridon, in einem Lösungsmittel wie einem Ether, z.B. tert-Butylmethylether, oder einem Kohlenwasserstoff, z.B. Toluol, oder einem halogenierten Kohlenwasserstoff oder einem Ester bevorzugt Methylenchlorid bzw. Essigester bei einer Temperatur von 0 bis 80°, bevorzugt 10 bis 25°. Unerwarteterweise wurde gefunden, dass bei der wässrigen Aufarbeitung die TMS-Schutzgruppe glatt gespalten wird und das Zielprodukt (X) ohne Isolierung des TMS-geschützten Zwischenproduktes in hoher Ausbeute und Reinheit erhalten werden kann.

In analoger Weise kann die Hydroxylamin-Gruppe unter Verwendung von Tetrahydopyranyl-hydroxylamin eingeführt werden. Die Abspaltung der Tetrahydropyranyl-Gruppe erfolgt zweckmässigerweise in einem Alkohol wie Methanol oder Ethanol in Gegenwart einer starken Säure wie einer Mineralsäure, vorzugsweise HCl, oder einer Sulfonsäure, vorzugsweise Methansulfonsäure oder para-Toluolsulfonsäure, bei Raumtemperatur.

Alternativ kann die Hydroxylamingruppe in c) unter Verwendung von Benzylhydroxylaminhydrochlorid und einem Aktivierungsmittel in einer Weise eingeführt werden, wie es vorstehend für die Amidbildung der Säure mit Piperidin beschrieben ist. Besonders bevorzugte Aktivierungsmittel sind Carbodiimide, z.B. Dicyclohexylcarbodiimid oder ein Isocyanid, z.B. tert-Butylisocyanid, bevorzugt 2-Morpholino-ethylisocyanid, in Gegenwart von stöchiometrischen oder katalytischen Mengen an Aktivestern bildenden Alkoholen, wie z.B. N-Hydroxysuccinimid, N-Hydroxybenzotriazol oder bevorzugt N-Hydroxy-2-pyridon. Die Anwendung und Herstellung solcher Isocyanide ist in EP 29 909 B1 beschrieben.

Die Abspaltung der Benzylgruppe erfolgt dann mittels Wasserstoff und einem Katalysator, wie vorstehend für die Debenzylierung einer Verbindung der Formel I, in der R¹ gleich Bz ist, beschrieben.

Weiterhin kann gemäss Absatz d) die direkte Überführung eines Esters in einer Verbindung der Formel I, worin R¹ geradkettiges (C₁-C₆)Alkyl, vorzugsweise Methyl, bedeutet, in das Benzylhydroxamat durch Aktivierung des O-Benzylhydroxylaminhydrochlorids mit einem Alkylmagnesiumhalogenid, bevorzugt i-Propylmagnesiumchlorid, in Gegenwart des Esters (I), in einem Lösungsmittel wie einem Ether, z.B. t-Butylmethylether oder, bevorzugt THF, bei einer Temperatur von -70° bis 50°, bevorzugt -20° bis 0°, erfolgen.

Die hydrogenolytische Abspaltung der Benzylgruppe zur Verbindung X erfolgt dann ebenfalls wie vorstehend für die Debenzylierung der Verbindung I, in der R¹ gleich Bz ist, beschrieben.

In einer bevorzugten Ausführungsform erfolgt die Herstellung von Verbindung (X) aus Verbindungen der Formel (I) nicht über den Schritt d) sondern über Schritt b) mit R¹ gleich tert-Butyl, gefolgt von der anschliessenden Umsetzung der Verbindung (IX) mit Trimethylsilylhydroxylamin.

Gemäss der vorstehend genannten Verfahrensschritten kann eine Verbindung der Formel X in einer höheren Ausbeute als nach den im Stand der Technik beschriebenen Verfahren erhalten werden.

Die neuen Zwischenverbindungen der Formel VII, VIII und II sind ebenfalls Gegenstand der vorliegenden Erfindung. Insbesondere sind dies:
(R)-4-[(S)-4-Benzyl-2-oxo-oxazolidon-3-yl]-3-cyclopentylmethyl-4-oxo-butansäure tert-butylester,
(R)-2-Cyclopentylmethyl-bernsteinsäure-4-tert-butylester und
(R)-3-Cyclopentylmethyl-4-oxo-4-piperidin-1-yl-butansäure tert-butylester.

### Beispiele

### Beispiel 1

Eine Lösung von 53.16 g (S)-4-Benzyl-2-oxazolidinon in 420 ml Tetrahydrofuran wurde bei -45° mit 197 ml 1.6 M Butyllithium in Hexan versetzt, anschliessend wurde eine Lösung von 49.18 g Cyclopentylpropionsäurechlorid in 105 ml Tetrahydrofuran zugegeben und die Lösung 1 Std. bei -45° gerührt. Das intermediär entstandene (S)-3-(3-Cyclopentyl-1-oxopropyl)-4-(phenylmethyl)-2-oxazolidinon wurde mit 286 ml einer 1.1 M Lithiumdiisopropylamid-Lösung in Tetrahydrofüran bei -45° behandelt, 1.5 Std. gerührt und anschliessend 64.38 g Bromessigsäure-tert-butylester in 60 ml Tetrahydrofuran zugegeben. Nach 4 Std. bei -45° wurde 600 ml halbgesättigte Ammoniumchlorid-Lösung zugegeben, die THF-Phase mit halbgesättigter Kochsalz-Lösung gewaschen, konzentriert und durch Zugabe von Hexan kristallisiert, wobei 94.5g (76%) reiner (de >99,9%) (R)-4-[(S)-4-Benzyl-2-oxo-oxazolidin-3-yl]-3-cyclopentylmethyl-4-oxo-butansäure tert-butylester, Smp. 113-119°, erhalten wurde. IR (KBr): 1768s, 1730s und 1695s (C=O).

### Beispiel 2

Zu einer Suspension von 78.5 g Oxazolidinon aus Beispiel 1 in 550 ml Isopropanol wurde bei 0° eine Lösung bestehend aus 36.7 g 35% Wasserstoffperoxid und 8.31 g Natriumhydroxid in 78 ml Wasser gegeben und 1 Std. bei 22° gerührt. Die Lösung wurde konzentriert, mit Natronlauge basisch gestellt und das ausgefallene (S)-4-Benzyl-2-oxazolidinon abfiltriert. Noch vorhandenes (S)-4-Benzyl-2-oxazolidinon wurde mit Methylenchlorid extrahiert, wonach total 32.68 g (98%) reines (S)-4-Benzyl-2-oxazolidinon, Smp. 86.5-88°, zurückgewonnen wurde. Die wässrige Phase wurde mit Salzsäure auf pH=3 gestellt und mit Isopropylacetat extrahiert. Die organischen Extrakte wurden gewaschen, getrocknet und eingedampft, wonach 47.79 g (99%) enantiomerenreiner (ee >99%) (R)-2-Cyclopentylmethylbernsteinsäure 4-tert-butylester als Oel erhalten wurde. IR (Film): 2700m, br. (COOH), 1733s und 1710s (C=O).

### Beispiel 3

Eine Suspension von 34.48 g Säure aus Beispiel 2 und 5.98 g N-Hydroxy-2-pyridon in 170 ml Isopropylacetat wurde bei 0° mit 12.03 g Piperidin und anschliessend mit einer Lösung von 30.53 g Dicyclohexylcarbodiimid in 92 ml Isopropylacetat versetzt und 16 Std. bei 22° gerührt. Die Suspension wurde mit 82 g 10% Essigsäure in Wasser behandelt, das Gemisch 4 Std. gerührt und abfiltriert. Die organische Phase wurde mit Natriumkarbonat und Wasser gewaschen, getrocknet, filtriert und eingeengt, wonach 43.89 g (100%) reiner, aus dem Oel kristallisierter (R)-3-Cyclopentylmethyl-4-oxo-4-piperidin-1-yl-butansäure tert-butylester (ee >99%), Smp. 38-40°, erhalten wurde. IR (Film): 1729s und 1641s (C=O).

### Beispiel 4

Zu einer Lösung von 8.76 g Kalium-bis-trimethylsilylamid in 80 ml Tetrahydrofuran wurde bei -60° eine Lösung von 10.07 g Ester aus Beispiel 3 in 50 ml Tetrahydrofuran getropft und 30 Min. bei -60° gerührt. Anschliessend wurde bei -60° eine Lösung von 7.76 g 3-Brommethyl-1,5,5-trimethyl hydantoin in 40 ml Tetrahydrofuran zugegeben und 30 Min. bei -60° gerührt. Das Reaktionsgemisch wurde mit halb-gesättigter Kochsalz-Lösung und mit verdünnter Salzsäure gewaschen, getrocknet, filtriert und eingeengt, wobei 15.11 g eines 9:1-Gemisches von 1-[2(R)-[1(R)-(tert-Butoxycarbonyl)-2-(3,4,4,-trimethyl-2,5-dioxo-1-imidazolidinyl)ethyl]-3-cyclopentylpropionyl]piperidin und 1-[2(R)-[1(S)-(tert-Butoxycarbonyl)-2-(3,4,4,-trimethyl-2,5-dioxo-1-imidazolidinyl)ethyl]-3-cyclopentylpropionyl] piperidin (78% Ausbeute an reiner anti-Verbindung) erhalten wurde, das ohne weitere Reinigung in die nächste Stufe eingesetzt wurde. Das Gemisch kann durch Chromatographie an Kieselgel mit Hexan/Essigester (1:1) getrennt werden.

### Beispiel 5

Eine Lösung von 15.11 g des 9:1 Gemisches aus Bsp 4. in 15 ml Essigsäure wurde bei 0° mit 15ml 33% Bromwasserstoff in Essigsäure versetzt und 4 Std. bei 0° gerührt. Die Lösung wurde mit Methylenchlorid verdünnt, mit Wasser gewaschen, die organischen Phasen getrocknet, filtriert und eingedampft. Der Rückstand wurde aus 26ml tert-Butylmethylether und 26 ml Hexan kristallisiert, wonach 6.90 g (70%) diastereomerenreines (de >98%) 1-[2(R)-[1(R)-Carboxy-2-(3,4,4,-trimethyl-2,5-dioxo-1-imidazolidinyl)ethyl]-3-cyclopentylpropionyl]piperidin (IX), Smp. 111-114°, erhalten wurde. IR (KBr): 1770m und 1715s (C=O).

### Beispiel 6

Eine Lösung von 1.78 g 1-[2(R)-[1(R)-(Methoxycarbonyl)-2-(3,4,4-trimethyl-2,5-dioxo-1-imidazolidinyl)ethyl]-3-cyclopentylpropionyl]piperidin (siehe Europäische Patentanmeldung mit der Anmeldenummer 97110942.6 (2. Juli 1997)) in 3 ml THF wurde mit einer Lösung von 0.69 g KOH in 6.1 ml Wasser versetzt, 5 Std. bei 0° und 10 Std. bei 40° kräftig gerührt. Das Gemisch wurde mit verdünnter Salzsäure auf pH=2 gestellt und mit 8 ml THF und 6 ml gesättigter Kochsalzlösung versetzt. Die THF-Phase wurde mit halbgesättigter Kochsalzlösung gewaschen, getrocknet und eingeengt. Der Rückstand enthielt 1.68 g zu ca. 95% reines 1-[2(R)-[1(R)-Carboxy-2-(3,4,4-trimethyl-2,5-dioxo-1-imidazolidinyl)ethyl]-3-cyclopentylpropionyl]piperidin IX . IR (KBr): 1769m und 1714s (C=O).

### Beispiel 7

Zu einer Suspension von 2.11 g 1-[2(R)-[1(R)-Carboxy-2-(3,4,4-trimethyl-2,5-dioxo-1-imidazolidinyl)ethyl]-3-cyclopentylpropionyl]piperidin IX aus Beispiel 5 oder 6 und 0.61 g N-Hydroxy-2-pyridon in 21 ml Methylenchlorid wurde bei 22° 0.78 g 2-Morpholino-ethylisocyanid gegeben und 3 Std. gerührt. Die Lösung wurde mit 0.58 g O-Trimethylsilyl-hydroxylamin versetzt und 2 Std. gerührt. Das Reaktionsgemisch wurde mit gesättigter NaHCO₃-Lösung und mit Wasser gewaschen und eingedampft. Der Rückstand wurde in 20 ml tert-Butylmethylether und 0.23 inl Wasser gelöst, 1 1/2 Std. bei 22° gerührt, die Suspension mit 10 ml Hexan verdünnt, abfiltriert und der Rückstand bei 22°/11 mbar getrocknet wobei man 1.82 g (83%) reines 1-[3-Cyclopentyl-2(R)-[1(R)-(hydroxycarbamoyl)-2-(3,4,4-trimethyl-2,5-dioxo-1-imidazolidinyl)ethyl]propionyl]piperidin (X) erhielt, MS (EI): 436 (40%).

### Beispiel 8

Zu einer Lösung von 1.38 g 1-[2(R)-[1(R)-Carboxy-2-(3,4,4-trimethyl-2,5-dioxo-1-imidazolidinyl)ethyl]-3-cyclopentylpropionyl]piperidin (IX) aus Beispiel 5 oder 6 in 13 ml Methylenchlorid wurde bei 0° nacheinander 0.74 g N-Ethylmorpholin, 0.60 g N-Hydroxybenzotriazol-hydrat und 0.75 g N-Ethyl-N'-(3-dimethylaminopropyl)-carbodiimid gegeben und 20 Min. bei 0° gerührt. Das Reaktionsgemisch wurde mit 0.45 g N-Ethylmorpholin und 0.63 g O-Benzylhydroxylamin-hydrochlorid versetzt, 30 Min. bei 0° und 17 Std. bei 22° gerührt. Die Lösung wurde mit 13 ml Methylenchlorid verdünnt, mit Natriumbikarbonatlösung und verdünnter Salzsäure gewaschen, getrocknet und eingeengt. Der Rückstand wurde aus Essigester/Hexan kristallisiert und das Kristallisat getrocknet, wobei man 1.26 g (73%) reines 1-[2(R)-[1(R)-(Benzyloxycarbamoyl)-2-(3,4,4-trimethyl-2,5-dioxo-1-imidazolidinyl)ethyl]-3- cyclopentylpropionyl]piperidin, Smp. 138-140°, erhielt.

Alternativ wurde zu einer Suspension von 10.54 g von Verbindung (IX) aus Bsp. 5 und 3.06 g N-Hydroxy-2-pyridon in 110 ml Methylenchlorid bei 22° 3.86 g 2-Morpholino-ethylisocyanid gegeben und 2 Std. gerührt. Die Lösung wurde mit 3.39 g O-Benzylhydroxylamin versetzt und 5 Std. gerührt. Das Reaktionsgemisch wurde mit verdünnter Salzsäure, NaHCO₃-Lösung und Wasser gewaschen, getrocknet und eingeengt. Der Rückstand liefert nach Umkristallisation aus Methylenchlorid/Hexan 11.19 g (85%) reines Benzylhydroxamat, Smp. 140-142°.

### Beispiel 9

Eine Lösung von 1.10 g 1-[2(R)-[1(R)-(Methoxycarbonyl)-2-(3,4,4-trimethyl-2,5-dioxo-1-imidazolidinyl)ethyl]-3-cyclopentylpropionyl]piperidin und 568 mg O-Benzylhydroxylamin-hydrochlorid in 7 ml THF wurde bei -20° mit 3.5 ml einer 2 M i-PrMgCl-Lösung in THF behandelt und nach 1 Std. bei - 20° erneut mit 1.7 ml des Grignard-Reagens versetzt. Nach weiteren 21/2 Std. bei -20° wurde mit Ammoniumchloridlösung versetzt und mit Methylenchlorid extrahiert. Die Extrakte wurden getrocknet und eingeengt. Der Rückstand wurde aus tert-Butylmethylether/Hexan kristallisiert und das Kristallisat getrocknet, wobei man ebenfalls 1-[2(R)-[1(R)-(Benzyloxy-carbamoyl)-2-(3,4,4-trimethyl-2,5-dioxo-1-imidazolidinyl)ethyl]-3-cyclopentylpropionyl]piperidin, Smp. 135-137° erhielt.

### Beispiel 10

Zur Debenzylierung wurde eine Suspension von 5.5 g 1-[2(R)-[1(R)-(Benzyloxycarbamoyl)-2-(3,4,4-trimethyl-2,5-dioxo-1-imidazolidinyl)ethyl]-3-cyclopentylpropionyl]piperidin aus Beispiel 8 oder 9 in 40 ml Ethanol und 1.7 g Pd/C (5%) bei 22° /1 bar während 4 h hydriert. Die Suspension wurde filtriert, das Filtrat vollständig eingeengt und der Rückstand aus Wasser kristallisiert wobei man 3.9 g (85%) reines 1-[3-Cyclopentyl-2(R)-[1(R)-(hydroxycarbamoyl)-2-(3,4,4-trimethyl-2,5-dioxo-1-imidazolidinyl)ethyl] propionyl]piperidin (X) erhielt, MS (EI): 436 (40%).

## Patentansprüche

1. Verfahren zur Herstellung von chiralen Bernsteinsäurederivaten der Formel (I) 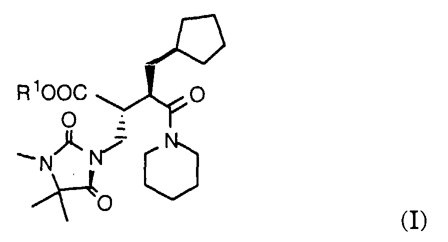 worin R¹ (C₁-C₆)Alkyl oder Benzyl bedeutet, dadurch gekennzeichnet, dass eine Verbindung der Formel (II) 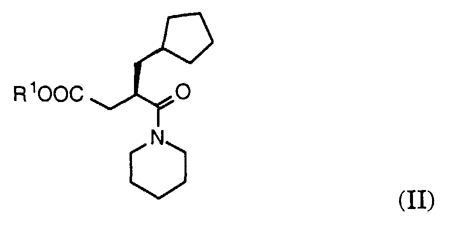 worin R¹ die vorstehend genannte Bedeutung hat,
mit einem Halogenhydantoin der Formel (III) 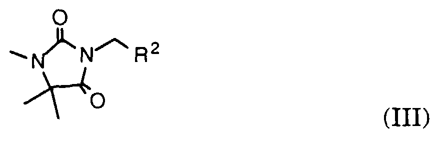 worin R² Chlor, Brom oder Jod ist,
in Gegenwart einer starken, enolatbildenden Kaliumbase umgesetzt wird.

2. Verfahren gemäss Anspruch 1, dadurch gekennzeichnet, dass als Kaliumbase KN(TMS)₂ verwendet wird.

3. Verfahren gemäss Anspruch 1 oder 2, dadurch gekennzeichnet, dass R¹ tert-butyl ist.

4. Verfahren gemäss einem der Ansprüche 1-3, dadurch gekennzeichnet, dass R² Brom ist.

5. Verfahren zur Herstellung von Bernsteinsäurederivaten gemäss einem der Ansprüche 1-4, dadurch gekennzeichnet, dass
(a) eine Verbindung der Formel (IV) 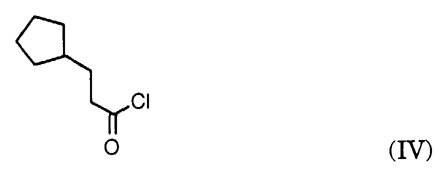 mit (S)-4-Benzyl-2-oxazolidinon zu (S)-3-(3-Cyclopentyl-1-oxopropyl)-4-(phenyl)-2-oxazolidinon (V) umgesetzt wird,
(b) das erhaltene Produkt mit einer Verbindung der Formel (VI) 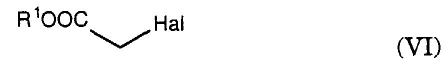 worin R¹ (C₁-C₆)Alkyl oder Benzyl und Hal Chlor, Brom oder Jod bedeutet, zu einer Verbindung der Formel (VII) 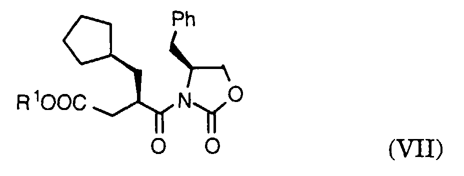 umgesetzt wird,
c) aus Verbindung VII unter Abspaltung von (S)-4-Benzyl-2-oxazolidinon eine Verbindung mit der Formel (VIII) erhält, 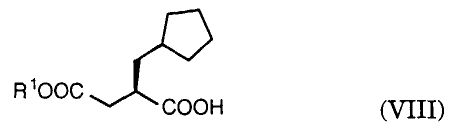
d) durch Umsetzung der Verbindung (VIII) mit Piperidin eine Verbindung der Formel (II) 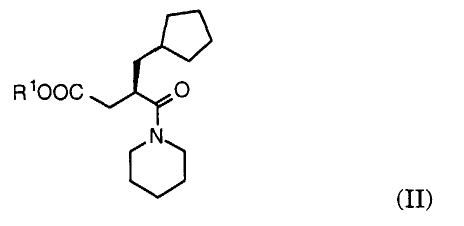 erhalten wird, und
e) die so erhaltene Verbindung der Formel (II) mit einem Halogenhydantoin der Formel (III) 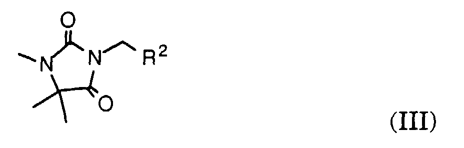 worin R² Chlor, Brom oder Jod ist,
gemäss einer der Ansprüche 1 bis 4 umgesetzt wird.

6. Verfahren nach Anspruch 5, dadurch gekennzeichnet, dass in Schritt b) R¹ in der Verbindung (VI) tert-Butyl ist.

7. Verfahren gemäss Anspruch 5 oder 6, dadurch gekennzeichnet, dass R² Brom ist.

8. Verfahren gemäss einem der Ansprüche 5 bis 7, dadurch gekennzeichnet, dass in Schritt c) die Abspaltung des (S)-4-Benzyl-2-oxazolidinon mittels H₂O₂ und NaOH in einem Gemisch von Wasser und einem Alkohol erfolgt.

9. Verfahren zur Herstellung einer Verbindung der Formel (X) 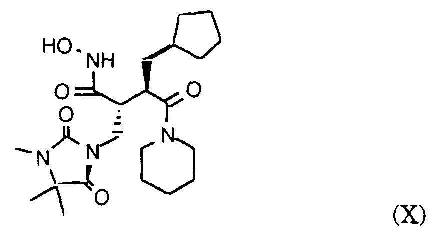 dadurch gekennzeichnet, dass
a) eine Verbindung der Formel (I) 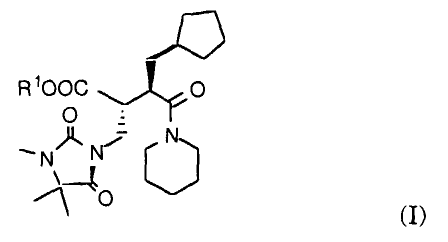 worin R¹ die in Anspruch 1 definierte Bedeutung hat, gemäss einem der Ansprüche 1-8 erhalten wird,
b) anschliessend durch Abspaltung der R¹ Gruppe die Verbindung mit der Formel (IX) 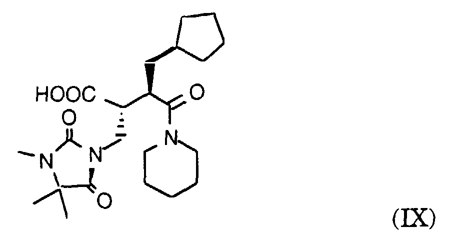 hergestellt wird, und
c) durch nachfolgende Einführung der Hydroxylamingruppe in der Verbindung der Formel (IX) die Verbindung der Formel (X) 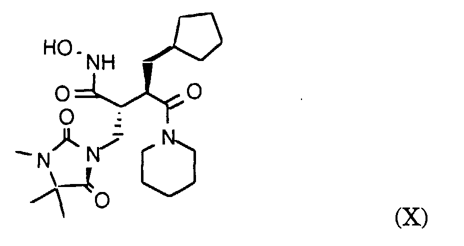 erhalten wird, oder
d) eine Verbindung der Formel (I), worin R¹ geradkettiges (C₁-C₆)Alkyl bedeutet,
wird mit einem mittels eines Alkylmagnesiumhalogenids aktivierten Benzylhydroxylaminhydrochlorids umgesetzt und anschliessend die Benzylgruppe hydrogenolytisch abgespalten, wobei ebenfalls Verbindung (X) erhalten wird.

10. Verfahren nach Anspruch 9, dadurch gekennzeichnet, dass wenn R¹ tert-butyl ist, die Abspaltung in Schritt b) mit einer Mineralsäure in einer Carbonsäure, vorzugsweise mit HBr/Essigsäure, erfolgt.

11. Verfahren nach Anspruch 9, dadurch gekennzeichnet, dass wenn R1 (C₁-C₆)Alkyl, ausser tert.Butyl oder eine sterisch ähnlich anspruchsvolle Alkylgruppe, ist, die Abspaltung in Schritt b) mit einem Alkali- oder Erdalkalimetallhydroxid erfolgt.

12. Verfahren nach Anspruch 9, dadurch gekennzeichnet, dass wenn R¹ Bz ist, die Abspaltung in Schritt b) hydrogenolytisch erfolgt.

13. Verfahren nach einem der Ansprüche 9 bis 12, dadurch gekennzeichnet, dass in Schritt c) bei Verbindung (IX) die Einführung der Hydroxylamingruppe
a) mittels Trimethylsilyl-hydroxylamin oder Tetrahydropyranylhydroxylamin und Abspaltung der Trimethylsilyl- bzw. Tetrahydropyranyl-Gruppe erfolgt, oder
b) mittels Benzylhydroxylamin-hydrochlorid und hydrogenolytischer Abspaltung der Benzylgruppe erfolgt.

14. Verbindungen der Formel (VII) 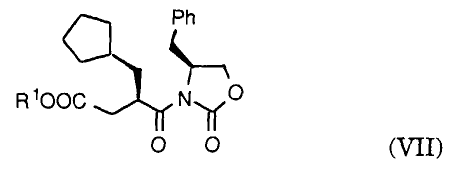 worin R¹ (C₁-C₆)Alkyl oder Benzyl bedeutet, insbesondere
(R)-4-[(S)-4-Benzyl-2-oxo-oxazolidon-3-yl]-3-cyclopentylmethyl-4-oxo-butansäure tert-butylester.

15. Verbindungen der Formel (VIII) 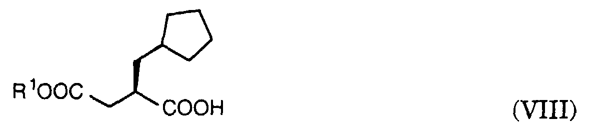 worin R¹ (C₁-C₆)Alkyl oder Benzyl bedeutet, insbesondere
(R)-2-Cyclopentylmethyl-bernsteinsäure-4-tert-butylester.

16. Verbindungen der Formel (II) 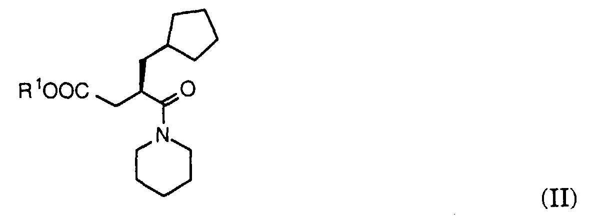 worin R¹ (C₁-C₆)Alkyl oder Benzyl bedeutet, insbesondere
(R)-3-Cyclopentylmethyl-4-oxo-4-piperidin-1-yl-butansäure tert-butylester

17. Verwendung eines Verfahrens gemäss einem der Ansprüche 1 bis 8 zur Herstellung von Verbindungen der Formel (I) 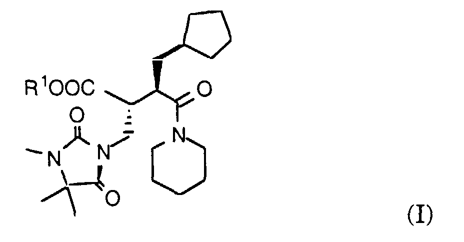 worin R¹ (C₁-C₆)Alkyl oder Benzyl bedeutet.

18. Verwendung eines Verfahrens gemäss einem der Ansprüche 1-13 zur Herstellung der Verbindung der Formel (X)

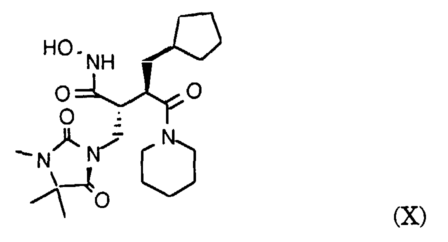

19. Verbindungen hergestellt gemäss einem der Ansprüche 1-13.
